# EUROPEAN PATENT APPLICATION

(11) **EP 4 215 521 A1**
(43) Date of publication of application: **26.07.2023**
(21) Application number: 21876963.6
(22) Date of filing: 28.09.2021
(51) Int. Cl.: C07D 221/14, C07D 401/06, C07D 401/12, A61K 31/496, A61P 35/02, A61P 35/00

(54) **FUSED RING DIIMIDE DERIVATIVE, PREPARATION METHOD THEREFOR AND USE THEREOF**

(30) Priority: 10.10.2020 CN 202011076343
(71) Applicant: Xiangbei Welman Pharmaceutical Co., Ltd, Hunan 410331 (CN); Guangzhou Xin-Chuangyi Biopharmaceutical Co., Ltd, Huangpu District Guangzhou, Guangdong 510535 (CN); Guangzhou Century Clinical Research Co., Ltd, Guangzhou, Guangdong 510535 (CN); Guangzhou Xin Chuang Yi Co., Ltd, Guangzhou, Guangdong 510535 (CN); Nanjing Kangfushun Pharmaceutical Co., Ltd, Nanjing, Jiangsu 210046 (CN)
(72) Inventor: SUN, Tianyu, Guangzhou, Guangdong 510620 (CN); CHU, Jiegen, Guangzhou, Guangdong 510620 (CN)
(74) Representative: Ladendorf, Oliver
(86) International application number: PCT/CN2021/121330
(87) International publication number: WO 2022/073445

(57) **Abstract**

Provided is a fused ring diimide derivative having a chemical structure of formula I. Further provided is a preparation method therefor. The fused ring diimide derivative has excellent anti-tumor activity, and the anti-proliferative activity on various cancer cells is significantly better than that of similar compounds.

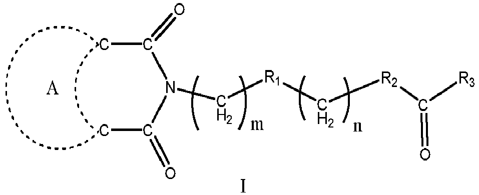

## Description

### TECHNICAL FIELD

The present disclosure relates to a fused ring diimide derivative, a preparation method and use thereof, and is in the field of medicinal chemistry.

### BACKGROUND

The diimide compound is an important class of substances in the field of medicinal chemistry with many pharmacological activities of interest.

When the diimide is fused with some fused rings, a unique planar structure with a greatly reduced molecular volume can be formed. In addition, the planar structure can be embedded between the base pairs of DNA double strands, resulting in unwinding of DNA double helix. Therefore, the diimide is of unique value in treating cell proliferative diseases, such as cancer. The fused rings fused with diimide can be various fused rings with conjugated unsaturated structures which can be carbocyclic or heterocyclic, such as naphthalene, anthracene and pyridocarbazole, etc. Many compounds with these structures have been reported to possess anti-tumor activities, but fewer of them have entered the clinical stage.

Amonafide is one of the representative compounds, in its molecular structure, the naphthalene ring is cycled with the diimide ring to form a planar structure. Amonafide has been reported to have cytotoxic activity against various cancers, such as colon cancer, lung cancer, gastric cancer, esophageal cancer and leukemia, etc. Antisoma has advanced Amonafide to clinical phase III for the treatment of acute myeloid leukemia, but the development was ultimately terminated due to poor efficacy.

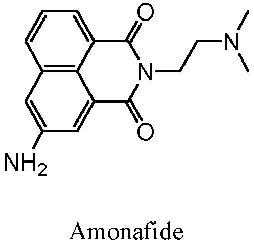

At present, other fused cyclic diimide compounds are still being investigated in the art for better potential active drugs, however, finding compounds with stronger activity than Amonafide remains difficult.

### SUMMARY

The present disclosure aims to provide a fused ring diimide compound with better properties.

In a first aspect, the present disclosure provides a compound having a fused ring diimide structure of formula I, 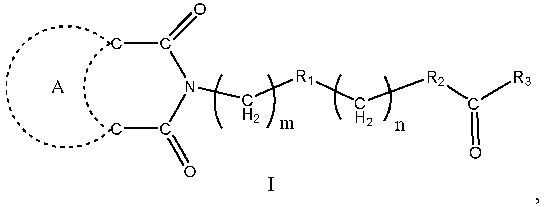
wherein, A is a optionally substituted fused ring, which is fused with diimide through 2 to 3 atoms;
m or n is 1, 2, 3, 4, 5, 6, 7, 8, 9 or 10;
R₁, R₂ or R₃ is selected from the group consisting of N, O and S; when R₁, R₂ or R₃ is N or S, R₁, R₂ or R₃ is optionally substituted, and R₁ optionally forms a ring together with R₂.

In some embodiments, in the formula I, when R₁, R₂ or R₃ is N, furthermore, R₁, R₂ or R₃ is optionally substituted.

In some embodiments, in the formula I, when R₁, R₂ or R₃ is S, R₁, R₂ or R₃ is optionally oxygenated to form sulfone or sulfoxide.

In some embodiments, in the formula I, A is a fused ring with a conjugated unsaturated structure.

In some embodiments, in the formula I, A is a fused ring formed by carbon rings, or a fused ring formed by carbon rings and heterocyclic rings.

In some embodiments, A is fused with diimide through 2 to 3 atoms to form a planar structure. Preferably, A is fused with diimide through 2 to 3 carbon atoms to form a planar structure.

In some embodiments, in the formula I, A is selected from the group consisting of fused bicyclic ring, fused tricyclic ring, fused tetracyclic ring and fused pentacyclic ring.

In some embodiments, in the formula I, A is selected from the group consisting of naphthalene, anthracene, phenanthrene, tetracene, chrysene, pyrene, perylene, quinoline, acridine, pyrrolopyridine, pyridocarbazole, naphtho[1,2-b]furan, benzimidazole and benzimidazole[1,2-C]quinoline. Preferably, A is selected from the group consisting of naphthalene, anthracene, phenanthrene, pyrene, perylene and naphtho[1,2-b]furan.

In some embodiments, in the formula I, A is optionally substituted by the substituent selected from the group consisting of alkyl, alkoxy, nitro, cyano, hydroxyl, amino, imino, tertiary amino and halogen, wherein alkyl and alkoxy can optionally be further substituted by hydroxyl or halogen. Preferably, A is substituted by the substituent selected from the group consisting of C1-C5 alkyl, C1-C5 alkoxy, nitro, cyano, imino, tertiary amino and halogen.

In some embodiments, in the formula I, m or n is 1, 2, 3, 4, 5 or 6.

In some embodiments, in the formula I, R₁ and R₂ are O or S.

In some embodiments, in the formula I, R₁ and/or R₂ are/is N.

In some embodiments, in the formula I, R₁ and R₂ are N.

In some embodiments, in the formula I, R₁ and/or R₂ are/is N, R₁ and/or R₂ are/is substituted by the substituent selected from the group consisting of alkyl, alkoxy, imino, tertiary amino, nitro and nitroso, wherein alkyl and alkoxy can optionally be further substituted by hydroxyl or halogen. Preferably, R₁ and/or R₂ are/is substituted by the substituent selected from the group consisting of C1-C5 alkyl, C1-C5 alkoxy, imino, tertiary amino, nitro and nitroso.

In some embodiments, in the formula I, R₁ is N, and is substituted by the substituent selected from the group consisting of alkyl, alkoxy, tertiary amino and nitro. Preferably, R₁ is substituted by the substituent selected from the group consisting of C1-C5 alkyl, C1-C5 alkoxy, tertiary amino and nitro.

In some embodiments, in the formula I, R₂ is N, and is substituted by the substituent selected from alkyl or alkoxy. Preferably, R₂ is substituted by C1-C5 alkyl or C1-C5 alkoxy.

In some embodiments, in the formula I, R₁ forms a ring together with R₂. Preferably, R₁ forms a six-membered ring together with R₂.

In some embodiments, in the formula I, R₃ is O or S, and R₃ is substituted by the substituent selected from the group consisting of alkyl, haloalkyl, alkoxy, alkoxyalkyl, nitro and nitroso, wherein the alkyl, alkoxy and alkoxyalkyl can optionally be further substituted by hydroxyl or halogen. Preferably, R₃ is substituted by the substituent selected from C1-C5 alkyl, C1-C5 haloalkyl, C1-C5 alkoxy, C1-C5 alkoxyC1-C5 alkyl, nitro and nitroso.

In some embodiments, in the formula I, R₃ is N, and is substituted by one or two substituents selected from the group consisting of alkyl, haloalkyl, alkoxy, alkoxyalkyl, nitro and nitroso. Preferably, R₃ is substituted by one or two substituents selected from the group consisting of C1-C5 alkyl, C1-C5 haloalkyl, C1-C5 alkoxy, C1-C5 alkoxyC1-C5 alkyl, nitro and nitroso.

In some embodiments, in the formula I, R₃ is N, and is substituted by two identical or different substituents selected from the group consisting of C1-C5 alkyl, C1-C5 haloalkyl, C1-C5 alkoxy, C1-C5 alkoxyC1-C5 alkyl, nitro and nitroso.

In some embodiments, in the formula I, R₁, R₂ and R₃ are N. Preferably, R₃ is substituted by one or two substituents selected from the group consisting of alkyl, haloalkyl, alkoxy, alkoxyalkyl, nitro and nitroso. Preferably, R₃ is substituted by one or two substituents selected from the group consisting of C1-C5 alkyl, C1-C5 haloalkyl, C1-C5 alkoxy, C1-C5 alkoxyC1-C5 alkyl, nitro and nitroso. When R₁, R₂ and R₃ are N and R₃ is substituted by one or two substituents, some preferred compounds of formula I have better properties, such as stronger activity.

In some embodiments, in the formula I, R₁, R₂ and R₃ are N. Preferably, R₃ is substituted by two identical or different substituents selected from the group consisting of C1-C5 alkyl, C1-C5 haloalkyl, C1-C5 alkoxy, C1-C5 alkoxyC1-C5 alkyl, nitro and nitroso. When R₁, R₂ and R₃ are N and R₃ is substituted by two substituents, some preferred compounds of formula I have better properties, such as stronger activity.

In some embodiments, in the formula I, the C1-C5 alkyl comprises methyl, ethyl, n-propyl, isopropyl, n-butyl, sec-butyl, isobutyl, tert-butyl, n-pentyl, etc., and the halogen comprises F, Cl, Br and I.

In a second aspect, the present disclosure provides a preparation method of the compound of formula I, comprising: 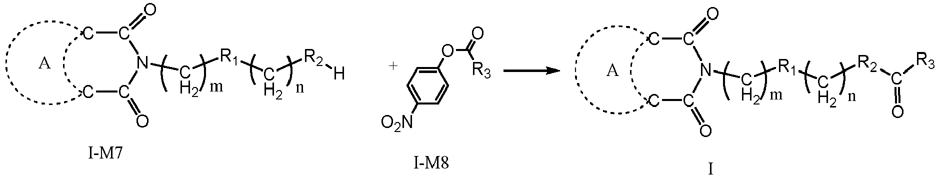 reacting a compound of formula I-M7 with a compound of formula I-M8 to obtain the compound of formula I, wherein A, m, n, R₁, R₂ and R₃ are as defined above.

In some embodiments, the compound of formula I-M7 reates with the compound of formula I-M8 in the presence of a base.

In some embodiments, the base is selected from the group consisting of sodium hydroxide, potassium carbonate, cesium carbonate and sodium phosphate.

The compound of formula I-M7 and the compound of formula I-M8 are commercially available or can be chemically synthesized.

In some embodiments, the compound of formula I-M7 can be prepared by the following method comprising: 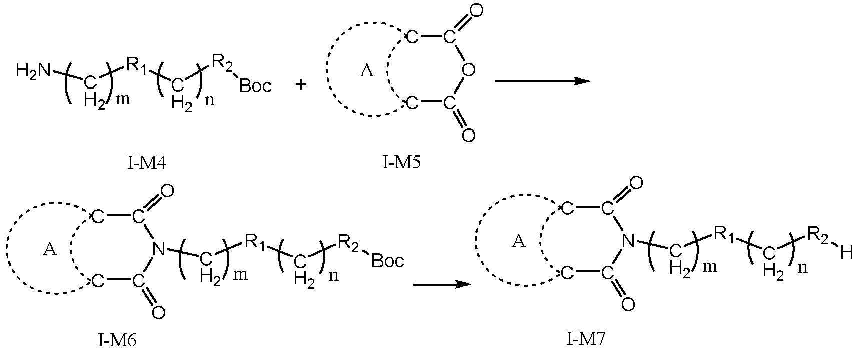 reacting a compound of formula I-M4 with a compound of formula I-M5 to obtain a compound of formula I-M6; and treating the compound of formula I-M6 in the presence of an acid to obtain the compound of formula I-M7, wherein, A, m, n, R₁ and R₂ are as defined above.

The compound of formula I-M4 is commercially available or can be chemically synthesized.

In some embodiments, the I-M4 can be prepared by the following method comprising: 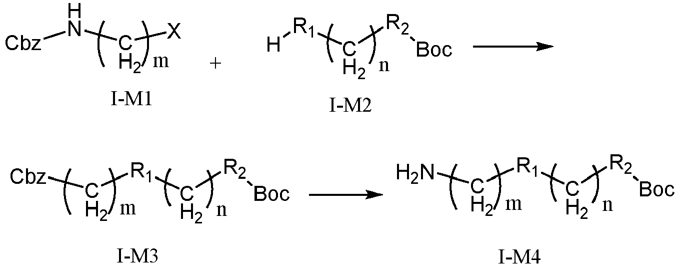 reacting a compound of formula I-M1 with a compound of formula I-M2 to obtain a compound of formula I-M3; and hydrogenating the compound of formula I-M3 to obtain the compound of formula I-M4, wherein X is halogen, and m, n, R₁ and R₂ are as defined above.

In a third aspect, the present disclosure provides use of the compound of formula I as described above in the preparation of a drug for treating a cell proliferative disease.

In some embodiments, the cell proliferative disease is cancer.

In a fourth aspect, the present disclosure provides a method for treating cell proliferative diseases, comprising administering a therapeutically effective amount of the compound of formula I to a subject in need thereof.

In some embodiments, the cell proliferative disease is cancer.

The fused ring diimide derivative provided by the present disclosure is a novel compound, which has been found to have excellent anti-tumor activity and have significant inhibitory effects on human colon cancer cells, lung cancer cells and leukemia cells. The anti-tumor activity of the fused ring diimide derivative is significantly better than that of similar compounds. Therefore, the fused ring diimide derivative has broad application prospects.

### DETAILED DESCRIPTION

The present disclosure is further described below with examples, and some preferred compounds will be exemplified. It should be noted that the examples are not intended to limit the compounds and technical effects of the present disclosure.

### Example 1: Compound I-01

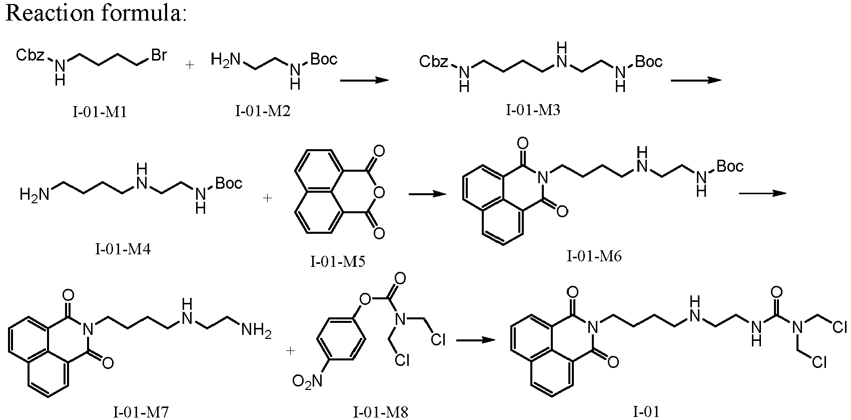

Compound I-01 was prepared according to the reaction formula, specifically as follows:
1.1 Preparation of intermediate I-01-M3: compound I-01-M2 (3.20 g, 20 mmol) was dissolved in 30 mL of dimethyl formamide (DMF), and then K₂CO₃ (4.16 g, 30 mmol), NaI (3.00 g, 20 mmol) and compound I-01-M1 (5.70 g, 20mmol) were added to the mixed solution successively, and the reaction mixture was stirred at 30°C overnight. After the reaction, purified water (120 ml) was added, and the reaction solution was extracted three times with ethyl acetate (180 ml), dried with anhydrous MgSO₄ and filtered. The mother liquor was concentrated and purified by column chromatography to obtain intermediate I-01-M3 (4.46 g, 12.2 mmol), with a yield of 61%.
1.2 Preparation of intermediate I-01-M4: to a mixture of intermediate I-01-M3 (4.46 g, 12.2 mmol) in a solution of methanol (20 mL) was added 10% Pd/C (0.3 g), hydrogen replacement was performed three times, the reaction mixture was stirred overnight under normal pressure, then filtered with diatomite. The mother liquor was concentrated to obtain colorless oily intermediate I-01-M4 (2.80 g, 12.1 mmol), with a yield of 99.2%.
1.3 Preparation of intermediate I-01-M6: intermediate I-01-M4 (2.80 g, 12.1 mmol), ethanol (55 ml) and compound I-01-M5 (2.40 g, 12.1 mmol) were added successively to a reaction flask, the reaction mixture was heated to 80°C and stirred for 2 hours, cooled, then concentrated and purified by column chromatography to obtain intermediate I-01-M6 (3.66 g, 8.9 mmol), with a yield of 73.6%.
1.4 Preparation of intermediate I-01-M7: intermediate I-01-M6 (3.66 g, 8.9 mmol) was dissolved into a 4% hydrogen chloride ethyl acetate solution (50 ml), the reaction mixture was stirred overnight at room temperature, then filtered and dried to obtain intermediate I-01-M7 (2.58 g, 8.3 mmol), with a yield of 93.3%.
1.5 Synthesis of compound I-01: compound I-01-M8 (2.50 g, 9 mmol), dichloromethane (27 ml) and triethylamine (2.02 g, 20 mmol) were added successively to a reaction flask and cooled to 0°C; intermediate I-01-M7 (2.58 g, 8.3 mmol) was added portion-wise, the reaction mixture was stirred at 0°C overnight, washed with water (15 ml), dried with anhydrous MgSO₄ and filtered. The mother liquor was concentrated and purified by column chromatography to obtain compound I-01(2.07 g, 4.6 mmol), with a yield of 55.4%. Product identification: ¹H-NMR (400MHz, d₆-DMSO) δ: 1.38-1.52(4H, m), 2.55(2H, t, J=6.8Hz), 2.78(2H, t, J=6.8Hz), 3.18(2H, t, J=6.5Hz), 3.26(2H, t, J=6.5Hz), 5.18(4H, s), 6.11(1H, m), 7.52(2H, m), 7.87(2H, dd, J=2.3, 8.1Hz), 7.93(2H, dd, J=2.3, 8.0Hz).

### Example 2: Compound 1-02

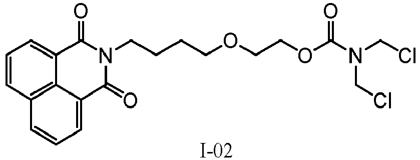

Compound I-02 was prepared by referring to the method of Example 1. Product identification: ¹H-NMR (400MHz, d₆-DMSO) δ: 1.33-1.48(4H, m), 3.20(2H, t, J=6.8Hz), 3.28(2H, t, J=6.8Hz), 3.58(2H, t, J=6.5Hz), 4.32(2H, t, J=6.5Hz), 5.12(4H, s), 7.48(2H, m), 7.89(2H, dd, J=2.3, 8.1Hz), 7.95(2H, dd, J=2.3, 8.0Hz).

### Example 3: Compound I-03

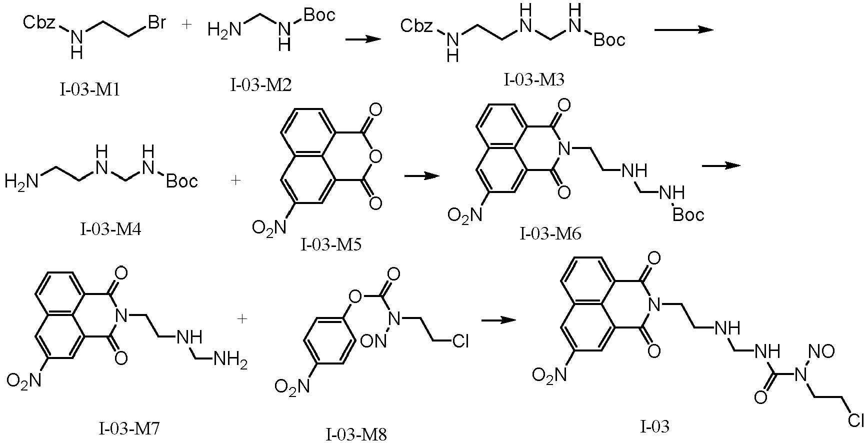

Compound I-03 was prepared according to the reaction formula, specifically as follows:
3.1 Preparation of intermediate I-03-M3: compound I-03-M2 (2.92 g, 20 mmol) was dissolved in 30 mL of DMF, and then K₂CO₃ (4.16 g, 30 mmol), NaI (3.00 g, 20 mmol) and compound I-03-M1 (5.14 g, 20 mmol) were added to the mixed solution successively, and the reaction mixture was stirred at 30°C overnight. After the reaction, purified water (120 ml) was added, and the reaction solution was extracted three times with ethyl acetate (180 ml), dried with anhydrous MgSO₄ and filtered. The mother liquor was concentrated and purified by column chromatography to obtain the intermediate I-03-M3 (3.75g, 11.6 mmol), with a yield of 58%.
3.2 Synthesis of intermediate I-03-M4: to a mixture of intermediate I-03-M3 (3.75 g, 11.6 mmol) in a solution of methanol (20 mL) was added 10% Pd/C (0.3 g), hydrogen replacement was performed three times, the reaction mixture was stirred overnight under normal pressure, then filtered with diatomite. The mother liquor was concentrated to obtain colorless oily intermediate I-03-M4 (2.19 g, 11.6 mmol), with a yield of 100%.
3.3 Synthesis of intermediate I-03-M6: intermediate I-03-M4 (2.19 g, 11.6 mmol), ethanol (50 ml) and compound I-03-M5 (2.82 g, 11.6 mmol) were added successively to a reaction flask, the reaction mixture was heated to 80°C and stirred for 2 hours, cooled, then concentrated and purified by column chromatography to obtain intermediate I-03-M6 (3.85 g, 9.3 mmol), with a yield of 80.2%.
3.4 Synthesis of intermediate I-03-M7: intermediate I-03-M6 (3.85g, 9.3 mmol) was dissolved into a 4% hydrogen chloride ethyl acetate solution (60 ml), the reaction mixture was stirred overnight at room temperature, then filtered and dried to obtain intermediate I-03-M7 (2.80 g, 8.9 mmol), with a yield of 95.7%.
3.5 Synthesis of compound I-03: compound I-03-M8 (2.73 g, 10 mmol), dichloromethane (35 ml) and triethylamine (2.02 g, 20 mmol) were added successively to a reaction flask and cooled to 0°C; intermediate I-03-M7 (2.80 g, 8.9 mmol) was added portion-wise, the reaction mixture was stirred at 0°C overnight, washed with water (25 ml), dried with anhydrous MgSO₄ and filtered. The mother liquor was concentrated and purified by column chromatography to obtain compound I-03 (2.60 g, 5.8 mmol), with a yield of 65.2%. Product identification: ¹H-NMR (400 MHz, d₆-DMSO) δ: 2.85(2H, t, J=6.8Hz), 3.18(2H, t, J=6.8Hz), 3.35(2H, t, J=6.5Hz), 3.80(2H, t, J=6.5Hz), 4.53(2H, s), 6.31(1H, m), 7.78(1H, m), 8.22(1H, dd, J=2.3,8.1Hz), 8.30(1H, dd, J=2.3, 8.1Hz), 8.78(1H, d, J=2.3Hz), 8.95(1H, d, J=2.3Hz).

### Example 4: Compound I-04

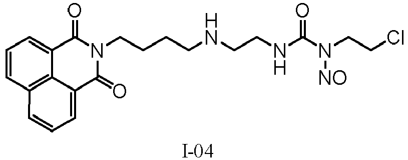

Compound I-04 was prepared by referring to the method of Example 3. Product identification: ¹H-NMR (400 MHz, d₆-DMSO) δ: 1.37-1.53(4H, m), 2.53(2H, t, J=6.8Hz), 2.79(2H, t, J=6.8Hz), 3.20(2H, t, J=6.5Hz), 3.29(2H, t, J=6.5Hz), 3.38(2H, t, J=6.5Hz), 3.62(2H, t, J=6.5Hz), 6.21(1H, m), 7.50(2H, m), 7.91(2H, dd, J=2.3, 8.0Hz), 7.98(2H, dd, J=2.3, 8.0Hz).

### Example 5: Compound I-05

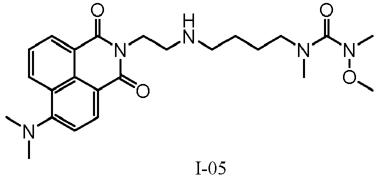

Compound I-05 was prepared by referring to the method of Example 3. Product identification: ¹H-NMR (400 MHz, d₆-DMSO) δ: 1.42-1.53(4H, m), 2.53(2H, t, J=6.8Hz), 2.63(3H, s), 2.78(3H, s), 2.83(2H, t, J=6.8Hz), 2.92(6H, s), 3.12(2H, t, J=6.5Hz), 3.33(2H, t, J=6.5Hz), 3.45(3H, s), 6.83(1H, d, J=8.0Hz), 7.48(1H, m), 7.78(1H, d, J=8.0Hz), 7.88(1H, dd, J=2.3, 8.0Hz), 7.98(1H, dd, J=2.3, 8.0Hz).

### Example 6: Compound I-06

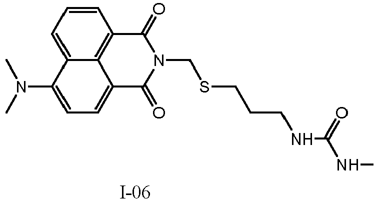

Compound I-06 was prepared by referring to the method of Example 3. Product identification: ¹H-NMR (400 MHz, d₆-DMSO) δ: 1.83(2H, m), 2.48(2H, t, J=6.8Hz), 2.68(3H, s), 2.91(6H, s), 3.12(2H, t, J=6.5Hz), 4.25(2H, s), 5.78(1H, m), 6.21(1H, m), 6.78(1H, d, J=8.0Hz), 7.45(1H, m), 7.77(1H, d, J=8.0Hz), 7.85(1H, dd, J=2.3, 8.0Hz), 7.95(1H, dd, J=2.3, 8.0Hz).

### Example 7: Compound I-07

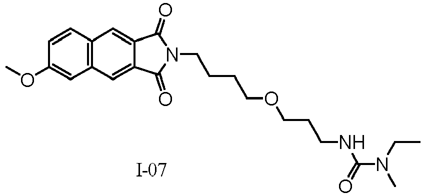

Compound I-07 was prepared by referring to the method of Example 3. Product identification: ¹H-NMR (400 MHz, d₆-DMSO) δ: 1.23(3H, t, J=6.8Hz), 1.45-1.75(6H, m), 2.65(3H, s), 3.06(2H, t, J=6.8Hz), 3.15(2H, q, J=6.8Hz), 3.25-3.38(4H, m), 3.58(2H, t, J=6.5Hz), 3.68(3H, s), 6.01(1H, m), 6.88(1H, d, J=2.3Hz), 7.02(1H, dd, J=2.3, 8.0Hz), 7.67(1H, dd, J=2.3, 8.0Hz), 8.42(1H, s), 8.49(1H, s).

### Example 8: Compound 1-08

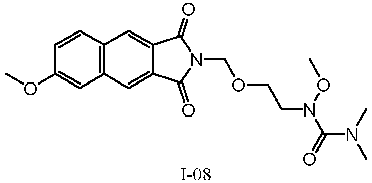

Compound I-08 was prepared by referring to the method of Example 3. Product identification: ¹H-NMR (400 MHz, d₆-DMSO) δ: 2.63(6H, s), 3.22(2H, t, J=6.8Hz), 3.36(3H, s), 3.63(2H, t, J=6.5Hz), 5.73(2H, s), 6.03(1H, m), 6.85(1H, d, J=2.3Hz), 7.01(1H, dd, J=2.3, 8.0Hz), 7.63(1H, dd, J=2.3, 8.0Hz), 8.38(1H, s), 8.51(1H, s).

### Example 9: Compound I-09

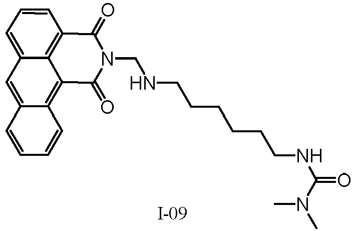

Compound I-09 was prepared by referring to the method of Example 3. Product identification: ¹H-NMR (400 MHz, d₆-DMSO) δ: 1.25-1.30(4H, m), 1.45-1.53(4H, m), 2.52(2H, t, J=6.8Hz), 2.68(6H, s), 3.08(2H, t, J=6.8Hz), 4.51(2H, s), 6.07(1H, m), 7.30(2H, m), 7.58(1H, m), 7.86(1H, dd, J=2.3, 8.0Hz), 7.95(1H, dd, J=2.3, 8.0Hz), 8.03(1H, dd, J=2.3, 8.0Hz), 8.20(1H, dd, J=2.3, 8.0Hz), 8.62(1H, s).

### Example 10: Compound I-10

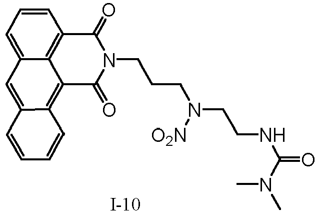

Compound I-10 was prepared by referring to the method of Example 3. Product identification: ¹H-NMR (400 MHz, d₆-DMSO) δ: 1.69(2H, m), 2.52(2H, t, J=6.8Hz), 2.68(6H, s), 2.82(2H, t, J=6.8Hz), 3.12(2H, t, J=6.8Hz), 3.28(2H, t, J=6.8Hz), 6.03(1H, m), 7.25(2H, m), 7.63(1H, m), 7.83(1H, dd, J=2.3, 8.0Hz), 7.98(1H, dd, J=2.3, 8.0Hz), 8.06(1H, dd, J=2.3, 8.0Hz), 8.25(1H, dd, J=2.3, 8.0Hz), 8.68(1H, s).

### Example 11: Compound I-11

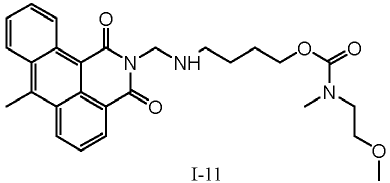

Compound I-11 was prepared by referring to the method of Example 3. Product identification: ¹H-NMR (400 MHz, d₆-DMSO) δ: 1.42-1.53(4H, m), 2.55(2H, t, J=6.8Hz), 2.70(3H, s), 2.88(3H, s), 3.10(2H, t, J=6.8Hz), 3.28(3H, s), 3.58(2H, t, J=6.8Hz), 4.12(2H, t, J=6.8Hz), 4.52(2H, s), 7.33(2H, m), 7.62(1H, m), 7.88(1H, dd, J=2.3, 8.0Hz), 8.02(1H, dd, J=2.3, 8.0Hz), 8.09(1H, dd, J=2.3, 8.0Hz), 8.22(1H, dd, J=2.3, 8.0Hz).

### Example 12: Compound I-12

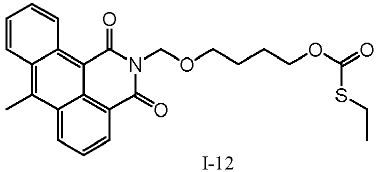

Compound I-12 was prepared by referring to the method of Example 3. Product identification: ¹H-NMR (400 MHz, d₆-DMSO) δ: 1.23 <3H, t, J=6.3Hz), 1.42(2H, m), 1.57(2H, m), 2.61(2H, t, J=6.8Hz), 2.70(3H, s), 2.92(2H, t, J=6.3Hz), 4.16(2H, t, J=6.7Hz), 4.76(2H, s), 7.52-7.65(3H, m), 7.80(1H, dd, J=2.3,8.0Hz), 7.91(1H, dd, J=2.3, 8.1Hz), 8.03(1H, dd, J=2.3, 8.2Hz), 8.27(1H, dd, J=2.3, 8.1Hz).

### Example 13: Compound I-13

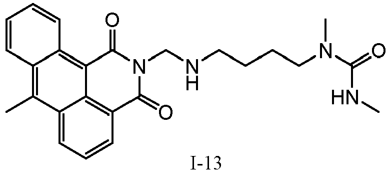

Compound I-13 was prepared by referring to the method of Example 3. Product identification: ¹H-NMR (400 MHz, d₆-DMSO) δ: 1.43-1.55(4H, m), 2.48(2H, t, J=6.8Hz), 2.58(3H, s), 2.62(3H, s), 2.78(3H, s), 3.12(2H, t, J=6.8Hz), 3.18(2H, t, J=6.8Hz), 4.42(2H, s), 7.38(2H, m), 7.65(1H, m), 7.83(1H, dd, J=2.3, 8.0Hz), 7.97(1H, dd, J=2.3, 8.0Hz), 8.12(1H, dd, J=2.3, 8.0Hz), 8.25(1H, dd, J=2.3, 8.0Hz).

### Example 14: Compound I-14

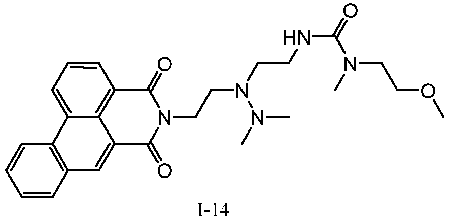

Compound I-14 was prepared by referring to the method of Example 3. Product identification: ¹H-NMR (400 MHz, d₆-DMSO) δ: 2.43(6H, s), 2.60(3H, s), 2.72-2.83(4H, m), 3.12(2H, t, J=6.8Hz), 3.18(2H, t, J=6.8Hz), 3.25(3H, s), 3.32(2H, t, J=6.8Hz), 3.69(2H, t, J=6.8Hz), 5.88(1H, m), 7.68-7.78(2H, m), 8.01(1H, m), 8.12(1H, dd, J=2.3, 8.0Hz), 8.36(1H, s), 8.48(1H, dd, J=2.3, 8.0Hz), 8.89(1H, dd, J=2.3, 8.0Hz), 9.21(1H, dd, J=2.3, 8.0Hz).

### Example 15: Compound I-15

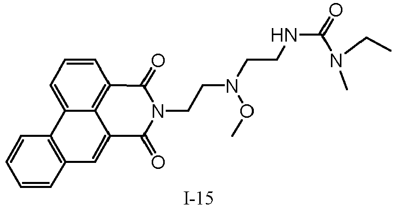

Compound I-15 was prepared by referring to the method of Example 3. Product identification: ¹H-NMR (400 MHz, d₆-DMSO) δ: 1.28(3H, t, J=6.8Hz), 2.68(3H, s), 2.72-2.83(4H, m), 3.12(2H, q, J=6.8Hz), 3.30(3H, s), 3.38(2H, t, J=6.8Hz), 3.46(2H, t, J=6.8Hz), 5.93(1H, m), 7.65-7.72(2H, m), 7.98(1H, m), 8.10(1H, dd, J=2.3, 8.0Hz), 8.33(1H, s), 8.45(1H, dd, J=2.3, 8.0Hz), 8.88(1H, dd, J=2.3, 8.0Hz), 9.18(1H, dd, J=2.3, 8.0Hz).

### Example 16: Compound I-16

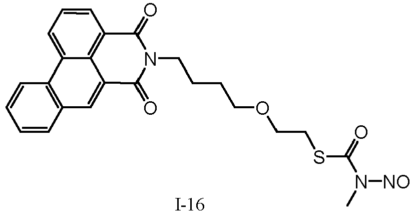

Compound I-16 was prepared by referring to the method of Example 3. Product identification: ¹H-NMR (400 MHz, d₆-DMSO) δ: 1.42-1.58(4H, m), 2.68(3H, s), 3.02(2H, t, J=6.8Hz), 3.18(2H, t, J=6.8Hz), 3.33(2H, t, J=6.8Hz), 3.98(2H, t, J=6.8Hz), 7.73-7.82(2H, m), 7.01(1H, m), 8.12(1H, dd, J=2.3, 8.0Hz), 8.33(1H, s), 8.47(1H, dd, J=2.3, 8.0Hz), 8.93(1H, dd, J=2.3, 8.0Hz), 9.23(1H, dd, J=2.3, 8.0Hz).

### Example 17: Compound I-17

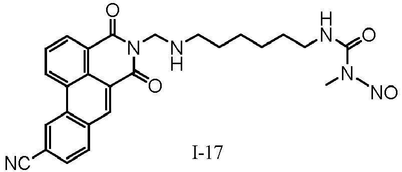

Compound I-17 was prepared by referring to the method of Example 3. Product identification: ¹H-NMR (400 MHz, d₆-DMSO) δ: 1.25-1.58(8H, m), 2.42(2H, t, J=6.8Hz), 2.66(3H, s), 3.12(2H, t, J=6.8Hz), 4.45(2H, s), 5.98(1H, m), 7.68(1H, dd, J=2.3, 8.0Hz), 8.02-8.12(2H, m), 8.35(1H, s), 8.47(1H, dd, J=2.3, 8.0Hz), 8.67(1H, d, J=2.3Hz), 9.12(1H, dd, J=2.3, 8.0Hz).

### Example 18: Compound I-18

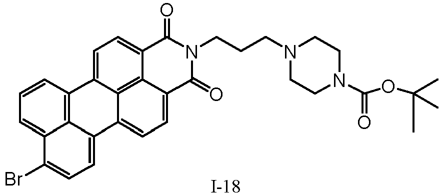

Compound I-18 was prepared by referring to the method of Example 3. Product identification: 1H-NMR (400 MHz, d₆-DMSO) δ: 1.43(9H, s), 1.69(2H, m), 2.45-2.76(6H, m), 3.01-3.11(4H, m), 3.25(2H, t, J=6.8Hz), 7.32-7.45(3H, m), 7.80-7.88(4H, m), 8.02(1H, d, J=8.1Hz), 8.10(1H, d, J=8.2Hz).

### Example 19: Compound I-19

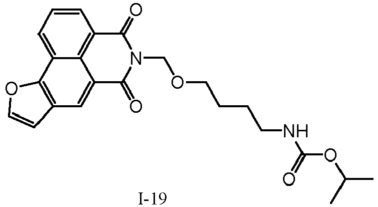

Compound I-19 was prepared by referring to the method of Example 3. Product identification: ¹H-NMR (400 MHz, d₆-DMSO) δ: 1.31(6H, d, J=6.8Hz), 1.43-1.55(4H, m), 2.92(2H, t, J=6.8Hz), 3.32(2H, t, J=6.8Hz), 5.45(2H, s), 6.38(1H, m), 6.68(1H, d, J=3.2Hz), 7.43(1H, d, J=3.2Hz), 7.48(1H, d, J=8.0Hz), 7.77(1H, dd, J=2.3, 8.0Hz), 7.86(1H, dd, J=2.3, 8.0Hz), 7.93(1H, s).

### Example 20: Compound I-20

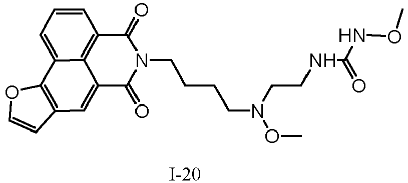

Compound I-20 was prepared by referring to the method of Example 3. Product identification: ¹H-NMR (400 MHz, d₆-DMSO) δ: 1.49-1.58(4H, m), 2.52(2H, t, J=6.8Hz), 2.78(2H, t, J=6.8Hz), 3.18(2H, t, J=6.8Hz), 3.32(3H, s) 3.45(2H, t, J=6.8Hz), 3.55(3H, s), 5.88(1H, m), 6.38(1H, m), 6.65(1H, d, J=3.2Hz), 7.42(1H, d, J=3.2Hz), 7.53(1H, d, J=8.0Hz), 7.69(1H, dd, J=2.3, 8.0Hz), 7.83(1H, dd, J=2.3, 8.0Hz), 7.88(1H, s).

### Example 21: Compound I-21

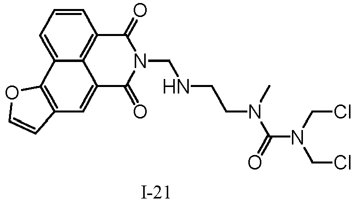

Compound I-21 was prepared by referring to the method of Example 3. Product identification: ¹H-NMR (400 MHz, d₆-DMSO) δ: 2.68(2H, s), 2.82(2H, t, J=6.8Hz), 3.26(2H, t, J=6.8Hz), 4.45(2H, s), 5.35(2H, s), 6.63(1H, d, J=3.2Hz), 7.48(1H, d, J=3.2Hz), 7.52(1H, d, J=8.0Hz), 7.73(1H, dd, J=2.3, 8.0Hz), 7.82(1H, dd, J=2.3, 8.0Hz), 7.91(1H, s).

### Example 22: Study on anti-tumor activity of the compound of formula I

Objective: To test the anti-proliferative activity of the compound of the present disclosure against various tumor cells.

Test compounds: Compounds I-01, I-02, I-03, I-04, I-05, I-08, I-10, I-12, I-15, I-17, I-18 and I-20; positive drug: Amonafide; and comparative compounds: 1d and 1. The Compound 1d refers to "1d" described in Journal of Cancer Molecules (2010,5 (2): p 41-47) and the Compound 1 refers to "Compond 1" described in Journal of Experimental Therapeutics and Oncology (2005,5: p 15-22), both Compounds 1d and 1 were prepared according to the method in the literature. The positive drug Amonafide is commercially available.

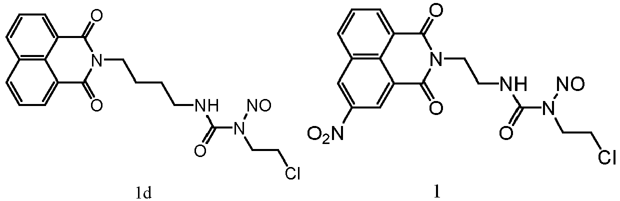

Test cells: human colon cancer cells (HT-29, COLO 205), human lung cancer cells (NCI-H460, A549) and human leukemia cells (HL-60, U-937).

Test method: each tumor cell was tested separately. Cells were incubated in medium at 37°C for 24 hours before use. The cells growing in an exponential growth phase were harvested and treated with trypsin to prepare a cell suspension, the cell suspension was centrifuged, then the cell pellet was resuspended in a small amount of fresh medium as a cell stock. The cell stock was diluted to required cell concentrations. The concentrations of each cell are as shown in Table 1:

**Table 1: Concentrations of Each Cancer Cells**

| Cell line | Cell concentration (cells count/mL) |
|---|---|
| HT-29 | 30000 |
| COLO 205 | 15000 |
| MCI-H460 | 15000 |
| A549 | 20000 |
| HL-60 | 30000 |
| U-937 | 30000 |

The test was carried out on 96-well plates; blank control groups, cell control groups and compound treatment groups were set up. In the blank control groups, only 10% PBS was added to each well, while in the cell control groups and the compound treatment groups, 100 µL of cells at the above concentration were added to each well, and then each well was filled with 200 µL of 10% PBS. The plates were placed in an incubator overnight. In the compound treatment groups, 100 µL of each test compound at different dilution concentrations was added to each well, the dilution concentrations of the compounds are as shown in Table 2.

**Table 2: Dilution Concentrations of Test Compounds**

| NO. | Concentrations of Compounds (µM) |
|---|---|
| 1 | 200 |
| 2 | 66.7 |
| 3 | 22.2 |
| 4 | 7.41 |
| 5 | 2.47 |
| 6 | 0.823 |
| 7 | 0.274 |
| 8 | 0.0914 |

After the addition of the drug to each well was completed, the plates were cultured in an incubator for 96 hours, 22 µL of resazurin sodium solution (Alarm blue, SIGMA R7017) was added to each well. The plates were returned to the incubator for another 4 hours of incubation and then shaken for 10 seconds after being taken out. The fluorescence value of each well were recorded at 530/590 nm.

The above operations were repeated three times.

The IC₅₀ value of each test compound was calculated by Prism7 software. IC₅₀ was divided into different grades according to values, that is, SS: < 1 µM; S: 1-5µM; A: 5-10 µM; B: 11-20µM; C: 21-50 µM; D: 51-100 µM; E: > 100 µM. The main results are as shown in Table 3.

**Table 3: IC₅₀ Grades of Inhibitory Effects of Compounds on Tumor Cells**

| Compounds | HT-29 | COLO205 | NCI-H460 | A549 | HL-60 | U-937 |
|---|---|---|---|---|---|---|
| I-01 | S | S | A | S | A | S |
| I-02 | A | A | A | A | A | A |
| I-03 | S | SS | A | S | S | A |
| I-04 | SS | S | A | A | S | S |
| I-05 | S | S | A | A | A | A |
| I-08 | B | A | A | A | A | A |
| I-10 | A | S | S | A | S | A |
| I-12 | A | A | A | A | B | B |
| I-15 | S | A | S | A | A | S |
| I-17 | S | A | S | S | A | S |
| I-18 | A | B | A | A | A | B |
| I-20 | A | A | A | S | A | A |
| 1d | C | C | D | C | C | B |
| 1 | C | C | B | A | B | C |
| Amonafide | B | A | B | S | A | B |

It can be seen from the test result that the anti-tumor activity of the compounds of the present disclosure is generally better than that of the positive drug Amonafide. As shown by the comparison between the compounds of the present disclosure and similar compounds, the overall inhibitory activity of the compounds of the present disclosure on tumor cells is significantly better than that of the Compound 1d and the Compound 1. Especially for colon cancer cells, the activity of some preferred compounds (such as Compounds I-03 and I-04) is 50 times more than that of similar compounds.

Both Compound 1d and Compound 1 are structurally modified compounds of Amonafide, they are obtained by modifying the alkylamino group on the right side of Amonafide, but their overall anti-tumor activity is inferior to that of Amonafide under the test condition of the present disclosure. The difference in activity between the compounds of formula I of the present disclosure and Compound 1d as well as Compound 1 suggests that, in the modification of alkylamino group on the right side of Amonafide, it is very important for the improvement of activity to introduce a heteroatom (R₁) and separate the heteroatom (R₁) from the heteroatom (R₂) connected to right-side carbonyl group with a carbon chain or a carbon ring.

Although the foregoing has been described the present disclosure in detail with the general description and specific embodiments, some modifications or improvements can be made by those skilled in the art on the basis of the present disclosure, and these modifications or improvements made without departing from the spirit of the present disclosure all fall within the scope of protection of the present disclosure as claimed.

## Claims

1. A fused ring diimide derivative of formula I, 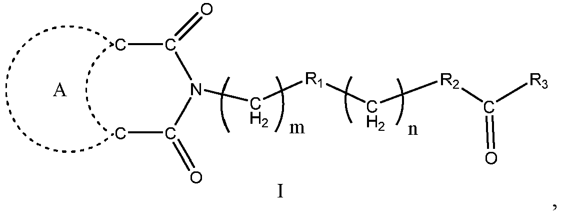
wherein, A is a optionally substituted fused ring, which is fused with diimide through 2 to 3 atoms;
m or n is 1, 2, 3, 4, 5, 6, 7, 8, 9 or 10; and
R₁, R₂ or R₃ is selected from the group consisting of N, O and S; when R₁, R₂ or R₃ is N or S, R₁, R₂ or R₃ is optionally substituted, and R₁ optionally forms a ring together with R₂.

2. The fused ring diimide derivative according to claim 1, wherein in formula I, when R₁, R₂ or R₃ is N, R₁, R₂ or R₃ is optionally substituted.

3. The fused ring diimide derivative according to claim 1, wherein in formula I, A is a fused ring with a conjugated unsaturated structure.

4. The fused ring diimide derivative according to claim 1, wherein in formula I, A is selected from the group consisting of fused bicyclic ring, fused tricyclic ring, fused tetracyclic ring and fused pentacyclic ring.

5. The fused ring diimide derivative according to claim 1, wherein in formula I, A is a fused ring formed by carbon rings, or a fused ring formed by carbon rings and heterocyclic rings.

6. The fused ring diimide derivative according to claim 1, wherein in formula I, A is selected from the group consisting of naphthalene, anthracene, phenanthrene, tetracene, chrysene, pyrene, perylene, quinoline, acridine, pyrrolopyridine, pyridocarbazole, naphtho[1,2-b]furan, benzimidazole and benzimidazole[1,2-C]quinoline.

7. The fused ring diimide derivative according to claim 6, wherein in formula I, A is selected from the group consisting of naphthalene, anthracene, phenanthrene, pyrene, perylene and naphtho[1,2-b]furan.

8. The fused ring diimide derivative according to claim 1, wherein in formula I, A is substituted by alkyl, alkoxy, nitro, cyano, amino, imino, tertiary amino or halogen.

9. The fused ring diimide derivative according to claim 8, wherein in formula I, A is substituted by C1-C5 alkyl, C1-C5 alkoxy, nitro, cyano, imino, tertiary amino or halogen.

10. The fused ring diimide derivative according to claim 1, wherein in formula I, m or n is 1, 2, 3, 4, 5 or 6.

11. The fused ring diimide derivative according to claim 1, wherein in formula I, R₁ and/or R₂ are/is O or S.

12. The fused ring diimide derivative according to claim 1, wherein in formula I, R₁ and/or R₂ are/is N.

13. The fused ring diimide derivative according to claim 12, wherein in formula I, R₁ and/or R₂ are/is substituted by alkyl, alkoxy, imino, tertiary amino, nitro or nitroso.

14. The fused ring diimide derivative according to claim 12, wherein in formula I, R₁ and/or R₂ are/is substituted by C1-C5 alkyl, C1-C5 alkoxy, imino, tertiary amino, nitro or nitroso.

15. The fused ring diimide derivative according to claim 12, wherein in formula I, R₁ is substituted by alkyl, alkoxy, tertiary amino or nitro.

16. The fused ring diimide derivative according to claim 12, wherein in formula I, R₂ is substituted by alkyl or alkoxy.

17. The fused ring diimide derivative according to claim 1, wherein in formula I, R₁ and R₂ are N, and R₁ forms a ring together with R₂.

18. The fused ring diimide derivative according to claim 17, wherein in formula I, R₁ forms a six-membered ring together with R₂.

19. The fused ring diimide derivative according to claim 1, wherein in formula I, R₃ is OorS.

20. The fused ring diimide derivative according to claim 19, wherein in formula I, R₃ is substituted by alkyl, haloalkyl, alkoxy, alkoxyalkyl, nitro or nitroso.

21. The fused ring diimide derivative according to claim 19, wherein in formula I, R₃ is substituted by C1-C5 alkyl, C1-C5 haloalkyl, C1-C5 alkoxy, C1-C5 alkoxyC1-C5 alkyl, nitro or nitroso.

22. The fused ring diimide derivative according to claim 1, wherein in formula I, R₃ is N.

23. The fused ring diimide derivative according to claim 22, wherein in formula I, R₃ is substituted by one or two substituents selected from the group consisting of alkyl, haloalkyl, alkoxy, alkoxyalkyl, nitro and nitroso.

24. The fused ring diimide derivative according to claim 22, wherein in formula I, R₃ is substituted by two identical or different substituents selected from the the group consisting of C1-C5 alkyl, C1-C5 haloalkyl, C1-C5 alkoxy, C1-C5 alkoxyC1-C5 alkyl, nitro and nitroso.

25. A preparation method of the fused ring diimide derivative of formula I, comprising: 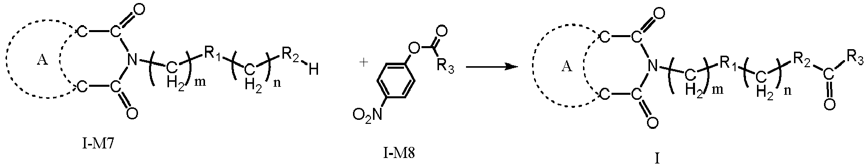 reacting a compound of formula I-M7 with a compound of formula I-M8 to obtain a compound of formula I according to the above reaction formula, wherein, A, m, n, R₁, R₂ and R₃ in the reaction formula are as defined in any one of claims 1 to 24.

26. The preparation method according to claim 25, wherein the compound of formula I-M7 reacts with the compound of formula I-M8 in the presence of a base.

27. The preparation method according to claim 25, wherein the compound of formula I-M7 is prepared by the following method comprising: 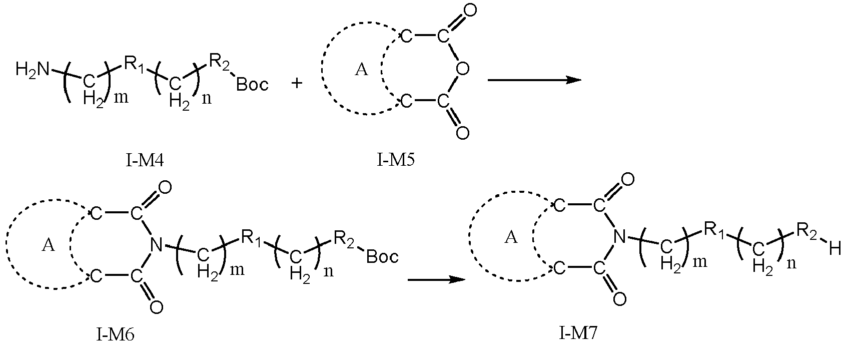 according to the reaction formula, reacting a compound of formula I-M4 with a compound of formula I-M5 to obtain a compound of formula I-M6; and treating the compound of formula I-M6 in the presence of an acid to obtain the compound of formula I-M7; wherein A, m, n, R₁ and R₂ in the reaction formula are as defined in any one of claims 1 to 24.

28. The preparation method according to claim 27, wherein the compound of formula I-M4 is prepared by the following method comprising: 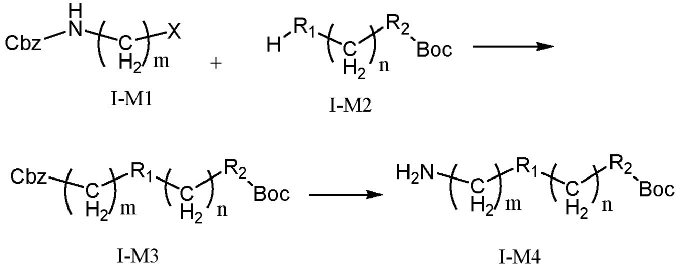 according to the reaction formula, reacting a compound of formula I-M1 with a compound of formula I-M2 to obtain a compound of formula I-M3; and hydrogenating the compound of formula I-M3 to obtain the compound of formula I-M4; wherein X in the reaction formula is halogen, and m, n, R₁ and R₂ are as defined in any one of claims 1 to 24.

29. Use of the fused ring diimide derivative according to any one of claims 1 to 24 in the preparation of a drug for treating a cell proliferative disease.

30. The use according to claim 29, wherein the cell proliferative disease is cancer.
